# FASCICULE DE BREVET EUROPEEN

(11) **EP 2 811 826 B1**
(45) Date de publication et mention de la délivrance du brevet: **03.04.2019**
(21) Numéro de dépôt: 13715303.7
(22) Date de dépôt: 12.03.2013
(51) Int. Cl.: A61L 2/12, A01M 21/04

(54) **PROCÉDÉ ET DISPOSITIF D'ÉRADICATION DE PLANTES ENVAHISSANTES ET PROLIFÉRANTES**
VERFAHREN UND VORRICHTUNG ZUR BESEITIGUNG INVASIVER, SICH AUSBREITENDER PFLANZEN
METHOD AND DEVICE FOR ERADICATING INVASIVE, PROLIFERATING PLANTS

(30) Priorité: 12.03.2012 FR 1252174
(43) Date de publication de la demande: 17.12.2014
(73) Titulaire: Géco Ingénierie, 30290 Laudun L'Ardoise (FR)
(72) Inventeur: ROURE, Frédéric, 30290 Laudun L'Ardoise (FR)
(74) Mandataire: Cornuejols, Georges
(86) Numéro de dépôt international: PCT/FR2013/050514
(87) Numéro de publication internationale: WO 2013/136007

(56) Documents cités:
- WO-A2-2008/057215
- NL-A- 8 900 730
- US-A1- 2002 090 268

## Description

### Domaine technique

La présente invention vise un procédé et un dispositif d'éradication de plantes envahissantes et proliférantes. Elle s'applique, en particulier, au nettoyage de berges de fleuves ou rivières et/ou à supprimer les plantes indésirables des matériaux apportés et/ou remblayés (terre végétale, compost).

### Etat de la technique antérieure

Des plantes exotiques envahissantes et proliférantes se propagent actuellement sur les berges des fleuves et rivières, notamment en Europe. Par exemple les Jussies (ludwigia sp.) et Renouées du Japon (Sackalines sp.), les Buddleya de Davidii, les Caulerpas, les Impatiences, les Ambroisies, font partie de ces plantes. L'envahissement qui s'en suit nuit à la biodiversité de ces milieux et détériorent l'habitat des riverains et des animaux qui y vivent ou vivent à leur proximité.

L'arrachage mécanique ou manuel de ces plantes est délicat. Leur prolifération impose une répétition des opérations d'arrachage. Surtout les déchets obtenus au cours de ces arrachages nécessitent un transport et des traitements complexes et coûteux.

On connaît des procédés et dispositifs de stérilisation de la surface du sol par utilisation de micro-ondes, par exemple ceux présentés dans les documents WO 2008/057215, NL 8 900 730 et US 2002/090268. Cependant, ces procédés ne permettent de traiter qu'une faible épaisseur du terrain et ne sont donc pas efficaces. De plus, ces procédés et dispositifs ne sont pas adaptés au traitement des plantes aquatiques et sont fortement perturbés par la présence de métaux à la surface du sol.

### Exposé de l'invention

La présente invention vise à remédier à tout ou partie de ces inconvénients en fournissant un dispositif et un procédé qui laissent sur place ces déchets rendus stériles et qui permettent l'implantation d'autres plantes, normalement présentes en Europe.

A cet effet, selon un premier aspect, la présente invention selon la revendication 1 vise un dispositif mobile d'éradication de plantes terrestres proliférantes et/ou envahissantes, qui comporte :
- un moyen de présentation des plantes à traiter et
- un moyen de génération d'ondes électromagnétiques pour stériliser les plantes dans la nappe de plantes.

Grâce à ces dispositions, les plantes envahissantes et proliférantes peuvent être directement stérilisées sur le lieu de leur collecte. De plus, les résidus, c'est-à-dire les plantes stérilisées, peuvent être laissées sur place et servir à nourrir les plantes de renaturation plantées à leur place. Il n'est donc nécessaire ni de transporter ces plantes envahissantes, ni de les entreposer hors-sol.

Selon l'invention, le moyen de présentation des plantes à traiter comporte un moyen de constitution d'une nappe de plantes à traiter.

On peut ainsi adapter l'épaisseur de la nappe à la puissance des ondes électromagnétiques.

Selon l'invention, le moyen de génération d'ondes électromagnétiques émet des micro-ondes.

Grâce à ces dispositions, les graines sont chauffées jusqu'à être désactivées.

Dans des modes de réalisation, le moyen de génération d'ondes électromagnétiques émet des micro-ondes dont la fréquence est comprise entre 500 MHz et 5 GHz.

Plus la longueur d'onde des micro-ondes est faible, plus ces micro-ondes sont pénétrantes.

Dans des modes de réalisation, le moyen de génération d'ondes électromagnétiques émet des micro-ondes dont la fréquence est comprise entre 500 MHz et 1 GHz.

L'utilisation de ces longueurs d'onde préférentielles permet de ne pas avoir à réduire l'épaisseur de la nappe de matière à traiter.

Selon l'invention, le moyen de génération d'ondes électromagnétiques est adapté à chauffer les graines des plantes à une température supérieure à 60 °C.

Grâce à ces dispositions, les graines sont chauffées jusqu'à être désactivées.

Dans des modes de réalisation, le dispositif d'éradication objet de la présente invention comporte, en outre, une trémie de réception des plantes et un moyen d'extraction de métaux et/ou de pierres.

Grâce à ces dispositions, les métaux, qui pourraient interagir avec les ondes électromagnétiques générées par le moyen de génération, sont extraits en amont de ce moyen de génération.

Dans des modes de réalisation, le dispositif objet de l'invention comporte, en outre, un moyen d'homogénéisation de l'épaisseur de la nappe de plantes à traiter, en amont du moyen de génération d'ondes électromagnétiques.

Grâce à ces dispositions, l'efficacité des ondes électromagnétiques est augmentée.

Le dispositif objet de la présente invention comporte, en outre, un moyen de broyage des plantes en amont du moyen de génération d'ondes électromagnétiques.

Grâce à ces dispositions, l'efficacité des ondes électromagnétiques est augmentée et les plantes sont tuées aussi par le broyage.

Dans des modes de réalisation, le dispositif objet de la présente invention comporte un moyen d'hydrocurage des plantes configuré pour sécher partiellement les plantes avant leur passage devant le moyen de génération d'ondes électromagnétiques.

On rappelle ici que l'hydrocurage consiste à séparer, au moins partiellement, l'eau des plantes à traiter. L'hydrocurage est une technique en amont du procédé de traitement thermique qui permet le pompage des plantes et des limons vers un poste d'égouttage. L'hydrocurage réalisé à l'aide d'une embarcation permet de pomper les plantes et leurs graines, les limons et les graviers avec de gros volumes d'eau

Selon un deuxième aspect, la présente invention selon la revendication 7 vise un procédé d'éradication de plantes terrestres proliférantes et/ou envahissantes, qui comporte :
- une étape de collecte des plantes,
- une étape de présentation des plantes à traiter et
- une étape de génération d'ondes électromagnétiques pour stériliser les plantes dans la nappe de plantes.

Les avantages, buts et caractéristiques du procédé objet de la présente invention étant similaires à ceux du dispositif objet de la présente invention, ils ne sont pas rappelés ici.

### Brève présentation des figures

D'autres avantages, buts et caractéristiques de la présente invention ressortiront de la description qui va suivre faite, dans un but explicatif et nullement limitatif, en regard des dessins annexés, dans lesquels :
- la figure 1 représente, schématiquement, un premier mode de réalisation particulier du dispositif d'éradication de plantes,
- la figure 2 représente, sous forme de logigrammes, des étapes d'un premier mode de réalisation particulier du procédé d'éradication objet de la présente invention,
- la figure 3 représente, sous forme de logigrammes, des étapes d'un deuxième mode de réalisation particulier du procédé d'éradication objet de la présente invention,
- la figure 4 représente, sous forme de logigrammes, des étapes d'un troisième mode de réalisation particulier du procédé d'éradication objet de la présente invention et
- la figure 5 représente, schématiquement, un deuxième mode de réalisation particulier du dispositif d'éradication de plantes.

### Exposé d'au moins un mode de réalisation de l'invention

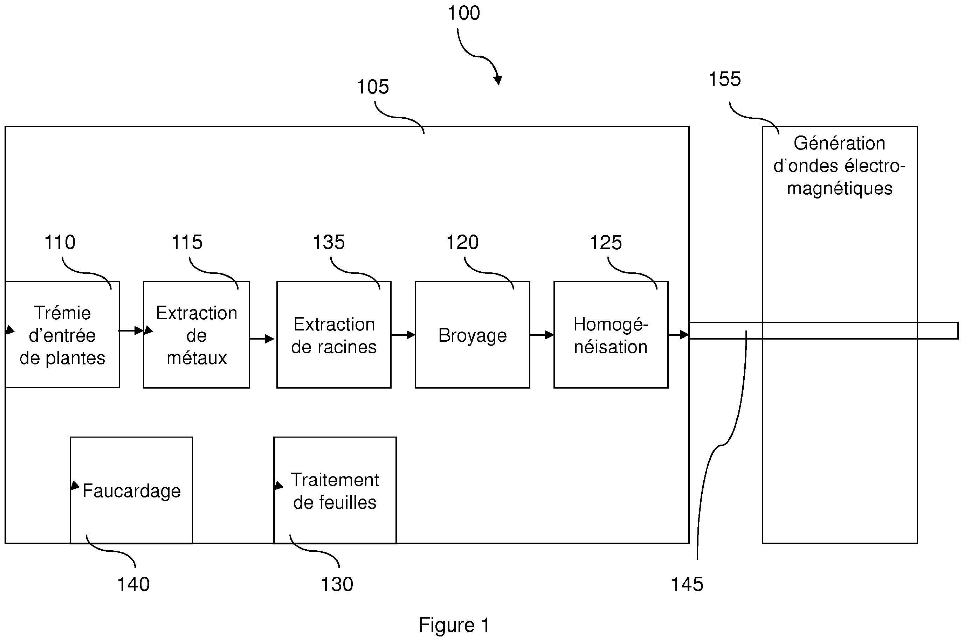

On observe, en figure 1, un dispositif 100 d'éradication de plantes qui comporte :
- un moyen 105 de constitution d'une masse de plantes à traiter et
- un moyen 155 de génération d'ondes électromagnétiques pour stériliser les plantes dans la nappe de plantes.

Le moyen 155 de génération d'ondes électromagnétiques émet des micro-ondes. Le moyen 155 de génération d'ondes électromagnétiques est adapté à chauffer et à maintenir en température de la masse de végétaux et, notamment, les graines des plantes à une température supérieure à 60 °C et, préférentiellement supérieure à 100 °C. Les graines sont ainsi chauffées jusqu'à être désactivées et, préférentiellement, l'ensemble des tissus de la plante sont tués.

Le moyen 105 de constitution d'une masse de plantes à traiter comporte, dans le cas où cette masse est mise en forme d'une nappe :
- une trémie 110 de réception des plantes,
- un moyen 115 d'extraction de métaux et/ou de pierres (pour extraire des macro-déchets et ferrailles, on met, par exemple, en oeuvre au moins un trommel et au moins un électroaimant) ; un séparateur granulométrique circulaire de type trommel permet d'éliminer les macro-déchets et gros blocs, débarrassés de tout fragment terreux ou organique ; ce trommel muni de différents calibres d'orifice permet un tri sélectif des matériaux selon leur blocométrie / granulométrie et leur poids,
- un moyen 120 de broyage des plantes qui homogénéise la masse végétale résiduelle,
- un moyen 125 d'homogénéisation de l'épaisseur d'une nappe de plantes à traiter, par exemple pour obtenir une couche de cinq à dix centimètres d'épaisseur et
- un convoyeur 145 qui achemine la nappe homogénéisée de plantes broyées jusqu'au moyen 155 de génération d'ondes électromagnétiques.

Optionnellement, le dispositif 100 objet de l'invention comporte, en outre :
- un moyen 130 de traitement de la végétation foliaire et des tiges des plantes, sans mise en oeuvre du moyen de génération d'ondes électromagnétiques et
- un moyen 135 d'extraction des systèmes racinaires, rhizomes ainsi que de la terre contaminée en amont du moyen de broyage 120 et/ou
- un moyen 140 de faucardage et transport de toute la partie végétative immergée pour traitement séparé sur la machine.

Comme on le comprend à la lecture de la description qui précède, les plantes envahissantes et proliférantes peuvent être directement stérilisées sur le lieu de leur collecte. De plus, les résidus, c'est-à-dire les plantes stérilisées, peuvent être laissées sur place et servir à nourrir les plantes de renaturation plantées à leur place. Il n'est donc nécessaire ni de transporter ces plantes envahissantes, ni de les entreposer hors-sol.

Les métaux, qui pourraient interagir avec les ondes électromagnétiques générées par le moyen de génération, sont extraits en amont de ce moyen de génération. L'efficacité des ondes électromagnétiques est ainsi augmentée.

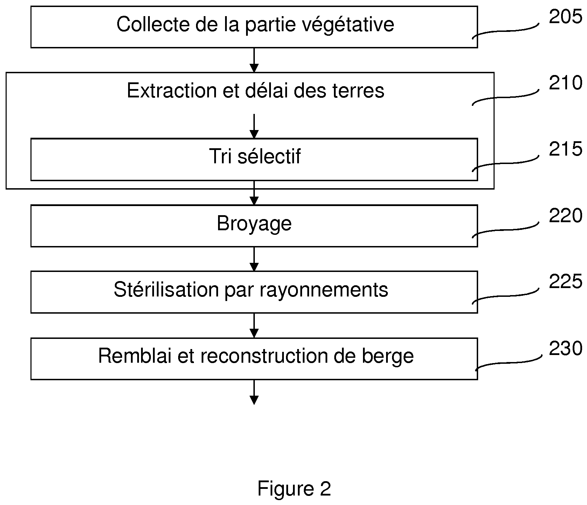

Dans son premier mode de réalisation, le procédé objet de la présente invention comporte d'abord, comme illustré en figure 2 :
- une étape 205 de mise à nue du site envahi pour collecter toute la partie végétative des plantes, et
- une étape 210 d'extraction et déblai des terres polluées.

L'étape 210 comporte une étape 215 de tri sélectif, pour extraire des macro-déchets et ferrailles, en mettant en oeuvre au moins un trommel et au moins un électroaimant.

On rappelle ici que le trommel est un système de tamisage à alimentation interne. Dans son principe de fonctionnement, le trommel est un tamis rotatif à alimentation interne. L'effluent est amené par pompage ou sous l'action de leur poids, à l'intérieur de la crépine. A débit identique, le tambour est plus long qu'un tambour de tamis rotatif. L'effluent a donc le temps de se répandre sur toute la surface de séparation, puis de traverser la crépine pour rejoindre la canalisation de sortie. Les déchets sont arrêtés à l'intérieur du cylindre et repoussés à l'opposé de l'alimentation par une vis sans âmes soudée au tambour. Ces refus de dégrillage sont alors évacués du caisson sous l'action de leur poids. Certains trommels sont équipés d'une rampe de rinçage. L'accès à la crépine est facilité par une trappe de visite équipée de contact sec pour une exploitation en toute sécurité. Le tambour est constitué d'un enroulement de fils à section triangulaire, sa longueur varie, par exemple, de 1200 à 1800 mm et son diamètre de 500 à 800 mm.

Puis, on réalise une étape 220 de broyage, pour homogénéiser la masse végétale résiduelle.

Au cours d'une étape 225, on réalise une stérilisation de la masse végétale broyée, par traitement thermique. On éradique ainsi les plantes envahissantes et proliférantes.

Au cours d'une étape 230, on remblaie et on reconstruit la berge avec la masse végétale broyée et stérilisée. On réalise ainsi une renaturation du site.

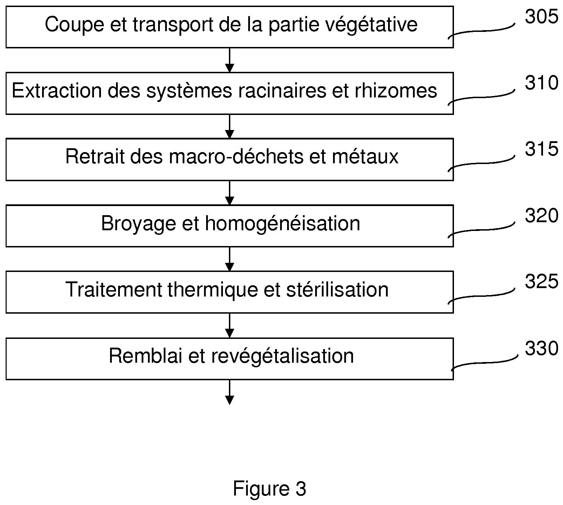

Dans son deuxième mode de réalisation, le procédé objet de la présente invention comporte d'abord, comme illustré en figure 3 :
- une étape 305 de coupe et transport de toute la partie végétative, pour évacuation de la végétation foliaire et des tiges pour traitement séparé sur la machine,
- une étape 310 d'extraction des systèmes racinaires, rhizomes ainsi que de la terre contaminée, par pelle mécanique pour l'extraction et chargement du broyeur mobile,
- une étape 315 de tri sélectif des macro-déchets par trommel et électro-aimant, au cours de laquelle les matériaux grossiers et les déchets métalliques sont rejetés et évacués,
- une étape 320 de broyage et homogénéisation des matériaux en couche de cinq à dix centimètres d'épaisseur, permettant une mise en nappe des matériaux à traiter,
- une étape 325 de traitement thermique et stérilisation, puis de rejet de l'ensemble des matériaux inertes, rhizomes broyés et inactifs et
- une étape 330 de remblai sur les zones d'emprunt, compactage du sol et re-végétalisation par les techniques du génie écologique.

Les étapes 305 et 310 peuvent comporter une étape d'hydrocurage.

On obtient ainsi un maintien hors du lit majeur des parties aériennes traitées et gravois hétérogènes douteux en zone de quarantaine ou repasse en tête de machine de traitement

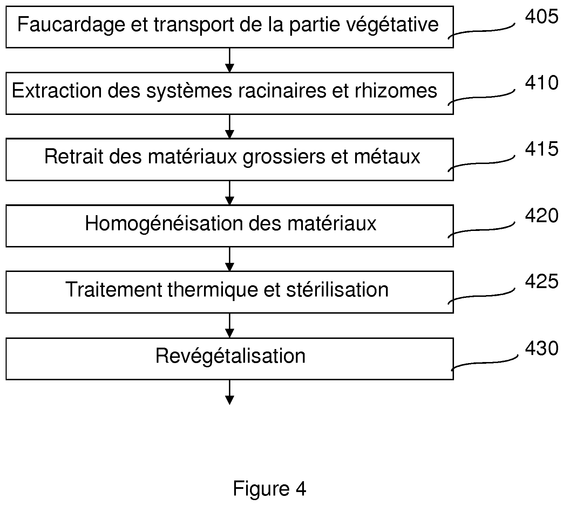

Dans son troisième mode de réalisation, le procédé objet de la présente invention comporte d'abord, comme illustré en figure 4 :
- une étape 405 de faucardage et transport de toute la partie végétative, au cours de laquelle on réalise l'évacuation de la végétation immergée par bateau faucard ou arrachage manuel pour traitement séparé sur la machine,
- une étape 410 d'extraction des systèmes racinaires, rhizomes ainsi que des limons et vases contaminés, par broyeur sous fluvial suivi de pompe d'évacuation des matériaux extraits (taille inférieure à dix centimètres),
- une étape 415 de tri sélectif des rhizomes et fragments de bois par trommel et électro-aimant, au cours de laquelle les matériaux grossiers et les déchets métalliques sont rejetés et évacués,
- une étape 420 d'homogénéisation des matériaux en couche de cinq à dix centimètres d'épaisseur ou bien en goulotte d'alimentation d'un tube de matériaux à traiter,
- une étape 425 de traitement thermique et de stérilisation, de maintien en température à la sortie de la machine puis de rejet de l'ensemble des matériaux inertes, rhizomes broyés et inactifs,
- une étape 430 de re-végétalisation par des hydrophytes endémiques et par les techniques du génie écologique.

Les étapes 405 et 410 peuvent comporter une étape d'hydrocurage.

On note que l'on maintient en andain des parties aériennes traitées et broyats hétérogènes douteux en zone de quarantaine.

On détaille, ci-après, les différentes étapes illustrées en figures 2 à 4.

Avant l'intervention de la machine d'éradication de plante exotique, le terrain doit être dépourvu de sa végétation foliaire et des tiges. Ceci pour ne pas encombrer la machine de matière végétale brute et augmenter son efficacité lors du traitement ultérieur du sol. Lors du débroussaillage, toutes les précautions doivent être prises pour ne pas propager la plante sur d'autre site. La matière végétale devra être conditionnée en fagots pour être engagée dans la trémie du broyeur. L'usage d'une pince de préemption sur le bras d'une pelle mécanique est l'outil le plus adapté.

Le matériel utilisé devra être nettoyé et dépourvu de tout fragment de plante avant une autre utilisation.

Les plantes exotiques peuvent avoir un système racinaire profond (cas de la Renouée du Japon), il faut donc creuser et extraire les terres polluées à une profondeur supérieure à un mètre sur certaines zones ou la plante est présente depuis plus de 10 ans. L'extraction ce fera à l'aide d'une pelle mécanique de plus de 8 tonnes. La terre polluée extraite doit être directement mis dans la machine à éradication.

Toutes les précautions doivent être prisent avant de réutiliser la pelle mécanique sur un autre site.

En ce qui concerne l'action d'éradication, la machine est utilisée pour l'alimentation de la trémie supérieure. La terre polluée est donc mise directement dans la trémie de la machine qui est constituée d'une première cellule de tri. Etant donné la nature hétérogène des matériaux extraits, il faut utiliser plusieurs méthodes de tri en fonction de la nature et de la taille des matériaux présent. Le substrat peut, en effet, être une ancienne zone de décharge ou bien un atterrissement de cours d'eau dynamique, composé de gros blocs. Ces contextes s'avèrent très favorables pour la colonisation des Renouées et les plus contraignantes au niveau technique de traitement des sols, les zones de plein pied avec une granulométrie inférieure à 10 cm étant les plus aisées dans le traitement des sols.

Puis la machine sépare les ferrailles. Un électroaimant monté sur un tapis d'extraction positionné au-dessus des déchets permet également d'éliminer les matériaux métalliques.

Un séparateur granulométrique circulaire de type trommel permet d'éliminer les macro-déchets et gros blocs, débarrassés de tout fragment terreux ou organique. Ce trommel muni de différents calibres d'orifice permet un tri sélectif des matériaux selon leur blocométrie / granulométrie et leur poids. Les gravats les plus grossiers (supérieurs à vingt centimètres selon les terrains) peuvent être rejetés directement après avoir été vérifiés visuellement de « l'absence » de toutes particules organiques, agglomérées dans des blocs d'argiles par exemple. Un opérateur contrôle donc visuellement cette première extraction.

La majorité des matériaux est ensuite amenée par tapis sur un broyeur de cailloux qui permet d'affiner tous les matériaux entrants (fraction de bois, blocs de moins de deux centimètres de diamètre, mottes de terres et rhizomes). Cette étape permet d'homogénéiser la fraction inerte mélangée avec les matières organiques à traiter et ainsi optimiser le traitement de celle-ci. Ces produits sont ensuite acheminés sur un tapis en lit de dix centimètres d'épaisseur vers la cellule de traitement a proprement dit. Le lit de produit homogène de dix centimètres d'épaisseur traverse un four à micro-ondes capable d'atteindre une température minimale de 100 °C sur toutes les molécules riches eau. Cette montée rapide en température provoque l'éclatement et la destruction des parois cellulosiques. Or, les cellules végétales, et donc des fragments de rhizomes, s'avèrent très riche en eau, représentant parfois plus de 90 % du poids frais.

La terre ainsi traitée est stérilisée de toute cellule végétale turgescentes et donc sans plantes exotiques. Les terres végétales d'apport et les composts peuvent, de même, être stérilisées en masse avant leur re-emploi et garantis « sans plantes exotiques ».

On donne, ci-dessous, des détails sur l'impact des micros ondes sur les cellules vivantes. Lorsque le four à micro-onde est en fonctionnement, il émet des rayonnements polarisés à une fréquence entre 500 GHz et 5 GHz, par exemple une fréquence de 2,4 GHz, et préférentiellement à une fréquence inférieure à 1 GHz, par exemple de 915 MHz. Le vecteur de polarisation de l'onde change à la fréquence des ondes émises. Les cellules des végétaux sont composées essentiellement d'eau. Or la molécule d'eau est une molécule dite polaire, ce qui signifie que les barycentres des charges négatives et positives ne sont pas confondus. De ce fait, il y a des endroits plus électronégatifs que d'autres. Cette particularité rend la molécule d'eau sensible aux variations de champ électrique produites par les micro-ondes. Concrètement, les molécules d'eau suivent la polarisation de l'onde, et ainsi change de sens plusieurs milliards de fois par seconde. C'est cette agitation des molécules qui fait chauffer, voire exploser, les cellules riches en eau. Les algues et les systèmes racinaires turgescents comme les rhizomes sont gorgés d'eau, avec des teneurs supérieures à 80 % de la biomasse.

La terre stérile et l'ensemble des rhizomes, broyés et inactifs sont rejetés.

Le procédé objet de la présente invention ne nécessite donc pas de stockage de rhizomes.

En ce qui concerne les actions postérieures de finition, les matériaux traités sont utilisés directement comme remblais dans les zones excavées précédemment. Ils sont compactés régulièrement en couche de 20 à 30 centimètres d'épaisseur pour limiter au maximum la reconquête de ces zones par de nouvelles invasions de plantes exotiques. La fraction terreuse supérieure remise en place est ensuite re-végétalisée par des méthodes de génie végétal, ceci afin de rendre au milieu sa stabilité et sa biodiversité.

On note que les travaux évitent tout risque d'aggravation de dissémination d'espèces envahissantes. L'usage de tous les moyens permettant de récupérer les brins de jussie flottants est systématique et rigoureux (filets pour les opérations mécanisées, géotubes, épuisette pour les interventions manuelles, bottes de paille éventuelles,...).

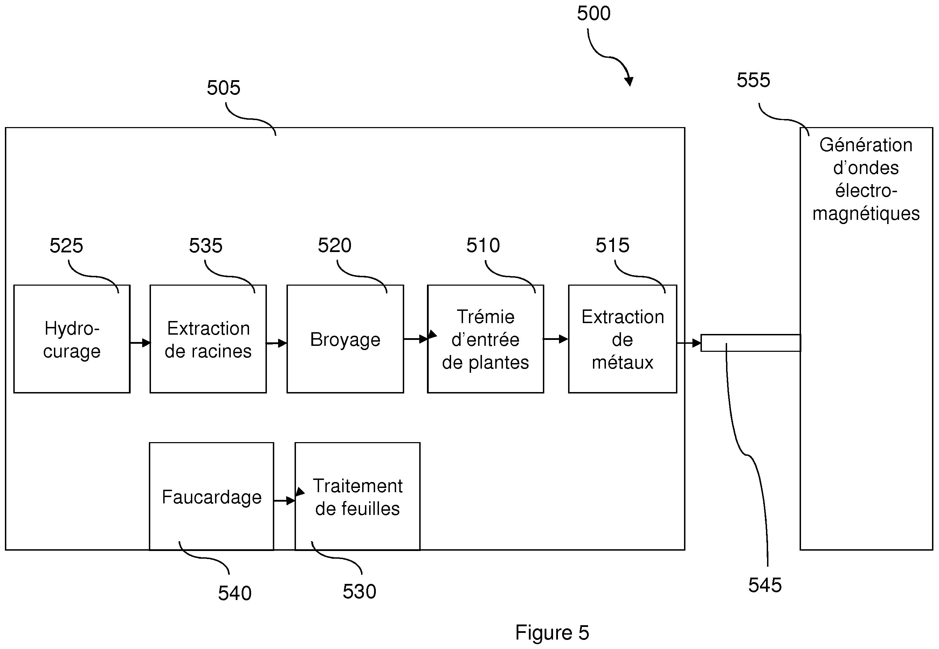

On observe, en figure 5, un dispositif 500 d'éradication de plantes qui comporte un un moyen 505 de présentation des plantes à traiter et un moyen 555 de génération d'ondes électromagnétiques pour stériliser les plantes dans la nappe de plantes. Ce moyen 555 de génération d'ondes électromagnétiques émet préférentiellement des micro-ondes et préférentiellement d'une fréquence inférieure à 1 GHz. Le moyen 555 de génération d'ondes électromagnétiques est adapté à chauffer les graines des plantes à une température supérieure à 60 °C et, préférentiellement supérieure à 100 °C. Les graines sont ainsi chauffées jusqu'à être désactivées et, préférentiellement, l'ensemble des tissus de la plante sont tués.

Le moyen 505 de présentation des plantes à traiter comporte :
- un moyen 525 d'hydrocurage des plantes configuré pour aspirer localement et transférer sur plateforme de traitement l'ensemble des plantes et leur substrat avant leur passage devant le moyen de génération d'ondes électromagnétiques,
- un moyen 535 d'extraction des systèmes racinaires, rhizomes ainsi que de la terre contaminée en amont d'un moyen de broyage 520 ; Les graines sont préférentiellement aussi extraites avec les matériaux fins, ou séparées par flottaison, avant traitement thermique,
- un moyen 520 de broyage de la partie extraite des plantes qui homogénéise la masse végétale résiduelle ; éventuellement, le moyen 520 fonctionne par dilacération (dans l'eau),
- une pompe de reprise et/ou convoyeur 545 qui achemine les plantes broyées jusqu'à la plateforme de traitement, moyen 555 de génération d'ondes électromagnétiques,
- un moyen 510 de transport des plantes jusqu'à terre et stockage dans une trémie d'entrée de plantes,
- un moyen 515 de dégrillage des matériaux extraits et déshydratation partielle.

Par exemple, le dégrillage comporte un convoyeur, de type convoyeur de vendange à vis sans fin, monté sur auge, percé sur tout le dessous pour faire s'écouler les matériaux de dimension inférieure à 5 mm. On peut aussi mettre en oeuvre un trömmel comme décrit à l'étape 215. Les matériaux solides tombent en haut du dispositif et les éléments fins, véhiculés par l'eau sont repris par pompage.

La déshydratation partielle est obtenue par des géotubes ou filtres bandes continus pour piéger les graines.

L'ensemble des matériaux solides (issus du dégrillage et des autres filtres) est envoyé dans le moyen 555 de génération d'ondes électromagnétiques, par exemple de micro-ondes.

Optionnellement, le dispositif 500 comporte, en outre :
- un moyen 540 de faucardage et transport de toute la partie végétative immergée pour traitement séparé sur la machine et/ou
- un moyen 530 de traitement direct de la terre d'apport ou de compost pouvant contenir des graines ou fragments de végétaux indésirables, AVEC mise en oeuvre du moyen de génération d'ondes électromagnétiques.

Comme on le comprend à la lecture de la description qui précède, les plantes envahissantes et proliférantes peuvent être directement stérilisées sur le lieu de leur collecte. De plus, les résidus, c'est-à-dire les matériaux stérilisés, peuvent être laissées sur place et servir à nourrir les essences plantées à leur place et sélectionnées pour leur usage (green et plantations paysagères).

Dans des variantes des différents modes de réalisation décrits ci-dessus, on élimine le moyen et l'étape de nappage de la matière à traiter. En particulier, l'utilisation de tubes verticaux, par exemple en téflon (marque déposée) et de fréquences d'ondes plus faibles, par exemple inférieures à 1 GHz permettent un traitement de la matière en profondeur.

Dans des variantes, on met en oeuvre un hydrocurage (séparation de l'eau des matières pompées dans la rivière ou le canal et sa préparation par un pré-nettoyage jusqu'au nettoyage final de telle sorte qu'elle puisse être utilisée de nouveau) des algues ou des plantes, permettant de les extraire puis de les sécher partiellement avant le traitement par micro-ondes. L'hydrocurage est une technique en amont du procédé de traitement thermique qui permet le pompage des plantes et des limons vers un poste d'égouttage. L'hydrocurage réalisé à l'aide d'une embarcation permet de pomper les plantes et leurs graines, les limons et les graviers avec de gros volumes d'eau.

## Revendications

1. Dispositif mobile (100, 500) d'éradication de plantes terrestres proliférantes et/ou envahissantes, qui comporte :
- un moyen (105, 505) de présentation des plantes à traiter comportant un moyen de constitution d'une nappe de plantes à traiter et
- un moyen (155, 555) de génération d'ondes électromagnétiques émettant des micro-ondes pour stériliser les plantes dans la nappe de plantes et qui est adapté à chauffer les graines des plantes à une température supérieure à 60°C dans la nappe de plantes et désactiver les graines et
- un moyen (120, 520) de broyage des plantes en amont du moyen de génération d'ondes électromagnétiques.

2. Dispositif (100, 500) selon la revendication 1, dans lequel le moyen (155, 555) de génération d'ondes électromagnétiques émet des micro-ondes dont la fréquence est comprise entre 500 MHz et 5 GHz.

3. Dispositif (100, 500) selon la revendication 2, dans lequel le moyen (155, 555) de génération d'ondes électromagnétiques émet des micro-ondes dont la fréquence est comprise entre 500 MHz et 1 GHz.

4. Dispositif (100, 500) selon l'une des revendications 1 à 3, qui comporte, en outre, une trémie (110) de réception des plantes et un moyen (115, 515) d'extraction de métaux et/ou de pierres.

5. Dispositif (100, 500) selon l'une des revendications 1 à 4, qui comporte, en outre, un moyen (125) d'homogénéisation de l'épaisseur de la nappe de plantes à traiter, en amont du moyen de génération d'ondes électromagnétiques.

6. Dispositif (500) selon l'une des revendications 1 à 5, qui comporte un moyen (525) d'hydrocurage des plantes configuré pour sécher partiellement les plantes avant leur passage devant le moyen (555) de génération d'ondes électromagnétiques.

7. Procédé d'éradication de plantes terrestres proliférantes et/ou envahissantes, qui comporte:
- une étape (205 à 215, 305 à 315, 405 à 415) de collecte des plantes,
- une étape (220, 320, 420) de présentation des plantes à traiter comportant une étape de constitution d'une nappe de plantes à traiter et
- une étape (225, 325, 425) de génération d'ondes électromagnétiques sous forme de micro-ondes pour stériliser les plantes dans la nappe de plantes et pour chauffer les graines des plantes à une température supérieure à 60°C dans la nappe de plantes et désactiver les graines et
- une étape de broyage des plantes en amont du moyen de génération d'ondes électromagnétiques.

8. Procédé selon la revendication 7, dans lequel, au cours de l'étape de génération d'ondes électromagnétiques (225, 325, 425), on génère des micro-ondes dont la fréquence est comprise entre 500 MHz et 5 GHz.

9. Procédé selon la revendication 8, dans lequel, au cours de l'étape de génération d'ondes électromagnétiques (225, 325, 425), on génère des micro-ondes dont la fréquence est comprise entre 500 MHz et 1 GHz.

## Patentansprüche

1. Mobile Ausmerzvorrichtung (100, 500) von übermäßig wachsenden und / oder lästigen Pflanzen, die umfasst:
- ein Mittel (105, 505) zum Einbringen der zu behandelnden Pflanzen, umfassend ein Bildungsmittel einer zu behandelnden Pflanzendecke und
- ein Mittel (155, 555) zum Erzeugen von elektromagnetischen Wellen, die Mikrowellen zum Sterilisieren der Pflanzen in der Pflanzendecke ausgeben und das geeignet ist, um die Samen der Pflanzen bei einer Temperatur von mehr als 60° C in der Pflanzendecke zu erhitzen und die Samen zu deaktivieren und
- ein Mittel (120, 520) zum Zermahlen der Pflanzen, das dem Mittel zum Erzeugen von elektromagnetischen Wellen vorgeschaltet ist.

2. Vorrichtung (100, 500) gemäß Anspruch 1, bei der das Mittel (155, 555) zum Erzeugen von elektromagnetischen Wellen Mikrowellen ausgibt, deren Frequenz zwischen 500 MHz und 5 GHz inbegriffen ist.

3. Vorrichtung (100, 500) gemäß Anspruch 2, bei der das Mittel (155, 555) zum Generieren von elektromagnetischen Wellen Mikrowellen ausgibt, deren Frequenz zwischen 500 MHz und 1 GHz inbegriffen ist.

4. Vorrichtung (100, 500) gemäß einem der Ansprüche 1 bis 3, das darüber hinaus einen Trichter (110) zur Aufnahme der Pflanzen und ein Mittel (115, 515) zur Extraktion von Metallen und / oder Steinen umfasst.

5. Vorrichtung (100, 500) gemäß einem der Ansprüche 1 bis 4, die darüber hinaus ein Mittel (125) zur Angleichung der Dicke der zu behandelnden Pflanzendecke umfasst, das dem Mittel zum Erzeugen von elektromagnetischen Wellen vorgeschaltet ist.

6. Vorrichtung (500) gemäß einem der Ansprüche 1 bis 5, die ein Mittel (525) zum Hochdruckreinigen der Pflanzen umfasst, das konfiguriert ist, um die Pflanzen teilweise vor ihrem Durchgang vor dem Mittel (555) zum Erzeugen von elektromagnetischen Wellen teilweise zu trocknen.

7. Ausmerzverfahren von übermäßig wachsenden und / oder lästigen Pflanzen, das umfasst:
- einen Schritt (205 bis 215, 305 bis 315, 405 bis 415) zum Sammeln der Pflanzen,
- einen Schritt (220, 320, 420) zum Einbringen der zu behandelnden Pflanzen, umfassend einen Schritt zum Bilden einer zu behandelnden Pflanzendecke und
- einen Schritt (225, 325, 425) zum Erzeugen von elektromagnetischen Wellen in Form von Mikrowellen zum Sterilisieren der Pflanzen in der Pflanzendecke und zum Erhitzen der Samen der Pflanzen auf eine Temperatur von mehr als 60° C in der Pflanzendecke und zum Deaktivieren der Samen und
- einen Schritt zum Zermahlen der Pflanzen, das dem Mittel zum Erzeugen von elektromagnetischen Wellen vorgeschaltet ist.

8. Verfahren gemäß Anspruch 8, bei dem im Verlauf des Schritts zum Erzeugen von elektromagnetischen Wellen (225, 325, 425) Mikrowellen generiert sind, deren Frequenz zwischen 500 KMhz und 5 GHz inbegriffen ist.

9. Verfahren gemäß Anspruch 8, bei dem im Verlauf des Schritts zum Erzeugen von elektromagnetischen Wellen (225, 325, 425) Mikrowellen generiert sind, deren Frequenz zwischen 500 KMhz und 1 GHz inbegriffen ist.

## Claims

1. Mobile device (100, 500) for eradicating proliferating and/or invasive terrestrial plants, comprising:
- a means (105, 505) for introducing the plants to be treated comprising a means for setting up a layer of plants to be treated, and
- a means (155, 555) for generating electromagnetic waves emitting microwaves for sterilizing the plants within the layer of plants and which is adapted to heat the seeds of the plants to a temperature above 60°C within the layer of plants and deactivate the seeds and
- a means (120, 520) for grinding the plants, which is arranged upstream from the means for generating electromagnetic waves.

2. Device (100, 500) according to claim 1, wherein the means (155, 555) for generating electromagnetic waves emits microwaves with a frequency between 500 MHz and 5 GHz.

3. Device (100, 500) according to claim 2, wherein the means (155, 555) for generating electromagnetic waves emits microwaves with a frequency between 500 MHz and 1 GHz.

4. Device (100, 500) according to one of claims 1 to 3, also comprising a hopper (110) for receiving the plants, and a means (115, 515) for removing metal and/or stones.

5. Device (100, 500) according to one of claims 1 to 4, also comprising a means (125) for making the thickness of the layer of plants to be treated uniform, upstream from the means for generating electromagnetic waves.

6. Device (500) according to one of claims 1 to 5, comprising a means (525) for water-jetting the plants, configured to partially dry the plants before they pass in front of the means (555) for generating electromagnetic waves.

7. Method for eradicating proliferating and/or invasive terrestrial plants, comprising:
- a step (205 to 215, 305 to 315, 405 to 415) of collecting plants;
- a step (220, 320, 420) of introducing the plants to be treated comprising a step of setting up a layer of plants to be treated, and
- a step (225, 325, 425) of generating electromagnetic waves in the form of microwaves for sterilizing the plants within the layer of plants and for heating the seeds of the plants to a temperature above 60°C within the layer of plants and deactivate the seeds and
- a step of grinding the plants, which is arranged upstream from the means for generating electromagnetic waves.

8. Method according to claim 7, wherein, during step of generating electromagnetic waves (225, 325, 425), microwaves with a frequency between 500 MHz and 5 GHz are generated.

9. Method according to claim 8, wherein, during step of generating electromagnetic waves (225, 325, 425), microwaves with a frequency between 500 MHz and 1 GHz are generated.
